# EUROPEAN PATENT SPECIFICATION

(11) **EP 2 859 906 B1**
(45) Date of publication and mention of the grant of the patent: **13.09.2017**
(21) Application number: 14186251.6
(22) Date of filing: 26.04.2011
(51) Int. Cl.: A61M 16/00, A61M 15/00, G01F 1/66, G06F 19/00

(54) **Drug delivery device**
Arzneimittelabgabevorrichtung
Dispositif d'administration de médicaments

(30) Priority: 26.04.2010 GB 201006901; 10.11.2010 GB 201019006
(43) Date of publication of application: 15.04.2015
(62) Divisional of application: 11718770.8
(73) Proprietor: Sagentia Limited, Harston, Cambridgeshire CB2 5GG (GB)
(72) Inventor: Harris, David, Harston, Cambridgeshire CB2 5GG (GB); Ansell, Iain, Harston, Cambridgeshire CB2 5GG (GB); Simpson, Richard, Harston, Cambridgeshire CB2 5GG (GB); Bonham, Peter, Harston, Cambridgeshire CB2 5GG (GB); Grubb, Scott, Harston, Cambridgeshire CB2 5GG (GB)
(74) Representative: MacDougall, Alan John Shaw

(56) References cited:
- WO-A1-90/10470
- WO-A1-2005/042076
- WO-A1-2007/101438
- US-A1- 2009 314 292
- RABBANI K S ET AL: "A novel gas flow sensor based on sound generated by turbulence and spirometry application", INSTRUMENTATION AND MEASUREMENT TECHNOLOGY CONFERENCE, 1997. IMTC/97. PROCEEDINGS. SENSING, PROCESSING, NETWORKING., IEEE OTTAWA, ONT., CANADA 19-21 MAY 1997, NEW YORK, NY, USA,IEEE, US, vol. 2, 19 May 1997 (1997-05-19), pages 1386-1388, XP010233794, DOI: 10.1109/IMTC.1997.612426 ISBN: 978-0-7803-3747-3

## Description

The present invention relates to drug delivery devices, parts thereof and methods. The invention has particular, although not exclusive, relevance to inhalers and to the use of sensing technology to monitor and measure use of various inhalers, such as dry powder inhalers (DPIs), metered dose inhalers (MDIs), nasal inhalers and nebulisers.

Inhalers are well known drug delivery devices. One of the main concerns about such drug delivery devices is the user's compliance with the intended usage. There is therefore a need to determine how the user is using the device. This can then be used to store usage information that can be subsequently transmitted to a physician or the like; or that can be used to control a user interface to provide feedback to the user, for example indicating correct or incorrect usage.

WO 2007/101438 discloses an inhaler device having an acoustic sensor and mentions that various parameters relating to the operation of the inhaler can be determined from the acoustic signal obtained from the acoustic sensor. The present invention aims to improve on the device disclosed in WO'438.

US 2009/314292 discloses interactive apparatus and methods for sensing and measuring real-time characteristic patterns of a subject's use of a dry powder inhalation system. The devices can be used in a wired or wireless communication mode to communicate with a display to assess the subject's usage of the inhalation system, to evaluate the performance of the inhalation system and/or to detect the characteristics profile of a dry powder formulation emitted from the inhalation system in use.

The invention aims to use acoustic sensing technology to accurately monitor and measure the use of a drug delivery device. The information collected can improve clinical trials and can be used to provide feedback to the user if desired.

The present invention provides processing circuitry for use in processing signals obtained from a microphone mounted on a body of a drug delivery device to determine operating conditions of the drug delivery device, the processing circuitry being characterised by: means for determining a frequency of a dominant frequency component or a harmonic thereof in the acoustic signal received from the microphone; and means for converting the determined frequency of the dominant frequency component or the harmonic thereof into a flow rate value using stored calibration data.

In one embodiment, the processing circuitry may track the dominant or harmonic frequency in the acoustic signal to determine a flow profile for at least a part of the inhalation.

The processing circuitry may further comprise: means for processing the signal obtained from the microphone to detect the timing of the delivery of a medicament during the inhalation. In this case, the device may further comprise: means for comparing the detected timing of said delivery relative to said flow profile with stored second calibration data to determine if the delivery of the medicament meets a desired delivery condition.

In one embodiment, the means for detecting the timing comprises means for detecting a change in the mean signal level of at least a portion of the signal obtained from the microphone. The portion is, in a preferred embodiment an upper frequency band of the signal obtained from the microphone.

The dominant or harmonic frequency may be detected by determining a spectrum of the signal obtained from the microphone and processing the spectrum to identify the dominant or harmonic frequency. Alternatively, the dominant or harmonic frequency may be determined using time domain based techniques, such as a bank of band pass filters and comparison circuits to compare the signal levels from the bank of filters; or by detecting zero crossings of the signal from the microphone.

The processing circuitry may further comprise means for determining the energy within the signal obtained from the microphone and for determining a flow rate measurement from the determined energy and stored calibration data. This energy based flow measurement may be used to check for anomalies in the tonal based flow measurement caused, for example, by the release of drug into the drug delivery device.

The present invention also provides a drug delivery system comprising: a drug delivery device having a body with a mouthpiece and a microphone mounted on the body for sensing sounds made by the drug delivery device during operation; and processing circuitry operable to process the signal obtained from the microphone to determine operating conditions of the drug delivery device.

In a preferred embodiment, the processing circuitry is mounted within the body of the drug delivery device. In this way, the device can provide real time feedback to the user, informing the user of correct or incorrect usage of the drug delivery device.

The drug delivery device may be a metered dose inhaler, a dry powder inhaler, a nebulizer or the like.

The invention also provides a computer program product comprising computer implementable instructions for causing a programmable processor device to become configured as the processing circuitry described above. The program product may include a CD, a DVD or other recording medium.

In order to aid in the understanding of the present invention, a number of exemplary embodiments will now be described in detail with reference to the accompanying figures in which:
Figure 1 is a cross sectional view illustrating the main components of a metered dose inhaler;
Figure 2 is a block diagram illustrating the main components of processing circuitry forming part of the inhaler device shown in Figure 1;
Figure 3 is a signal diagram illustrating the way in which a windowing function module of the processing circuitry shown in Figure 2 extracts windows or blocks of samples from an input audio signal;
Figure 4 is a plot illustrating the way in which the energy within the sensed acoustic signal varies with the flow rate of air through the inhaler of Figure 1;
Figure 5 illustrates a flow profile obtained using the processing circuitry shown in Figure 2 and illustrating peaks corresponding to potential firings of the metered dose inhaler;
Figure 6 illustrates the way in which the spectrum of the sensed acoustic signal changes with different flow rates and illustrating the spectrum of a sound obtained due to the firing of the metered dose inhaler;
Figure 7 is a flow chart illustrating the processing steps performed by the circuitry shown in Figure 2 in order to make flow measurements and to detect firing of the metered dose inhaler;
Figure 8 is a flow chart illustrating the processing steps performed by the circuitry shown in Figure 2 to process the measurements obtained using the steps illustrated in Figure 7 in order to determine whether or not the inhaler is used properly and if the firing occurred at the correct timing;
Figure 9 is a block diagram illustrating alternative processing circuitry that may be used with the inhaler device illustrated in Figure 1;
Figure 10 is an exploded partial view of a dry powder inhaler according to an embodiment;
Figure 11 is a block diagram illustrating processing circuitry forming part of the inhaler device shown in Figure 10;
Figure 12 illustrates a spectrum obtained by an FFT module forming part of the circuitry shown in Figure 11;
Figure 13 illustrates spectrums obtained from the FFT module for different flow rates;
Figure 14 illustrates the way in which a peak frequency component varies with flow rate;
Figure 15 illustrates a flow profile obtained using the processing circuitry shown in Figure 11 and illustrating peaks corresponding to potential firings of the BAM mechanism; and
Figure 16 is a plot illustrating a difference in spectrums obtained for the same flow rate with and without actuation of a breath activation mechanism.

### Metered dose inhaler

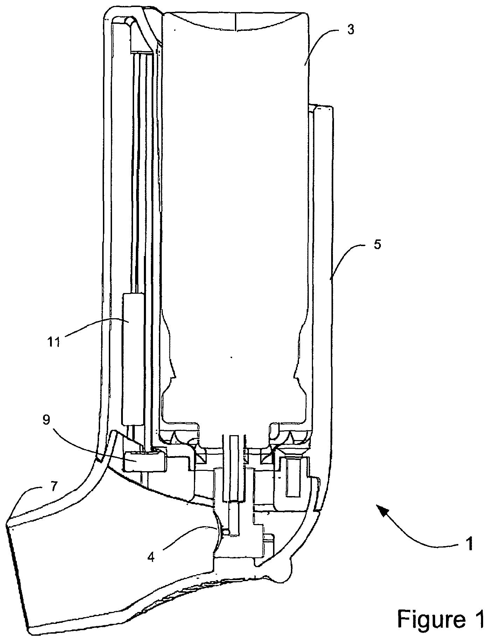

Figure 1 illustrates the form of a metered dose inhaler. The inhaler 1 includes a canister 3 which holds a medicament to be delivered; a metering valve 4 which allows a metered quantity of the medicament to be dispensed with each actuation; and an actuator 5 housing the canister 3 and having a mouthpiece 7, which allows the patient to operate the device and directs the aerosol into the patient's lungs. To use the inhaler, the patient typically presses down on top of the canister 3 which releases a single metered dose of the medicament which is inhaled by the user via the mouthpiece 7.

In this case, the inhaler includes a microphone 9 that is preferably positioned upstream of the aerosol (i.e. upstream of the metering valve 4) within an air channel defined by the inhaler body. This means that the aerosolised medicament does not come into contact with the microphone 9 and the addition of the microphone 9 to the inhaler device is non-invasive. The microphone 9 preferably sits flush with the inner surface of the air channel and thus does not affect the air flow through the channel. A condenser microphone is typically used.

As will be explained in more detail below, acoustic signals from the microphone 9 are analysed using a set of algorithms that are tailored for a particular inhaler type. The algorithms can be run on a microprocessor 11 such as a PIC or other programmable device such as a bespoke ASIC device.

In this case, the algorithms for the metered dose inhaler allow determination of volumetric flow rates through the inhaler by analysing the energy in the acoustic signal. The algorithms described below can also detect the firing or actuation of the metered dose inhaler canister 3, even at high air flow rates.

The information obtained by the processing circuitry (microprocessor 11) can then be used, for example, as a training aid for the user or for providing feedback to clinicians in clinical trials or to doctors or other physicians for patient monitoring. For example, the inhaler device 1 illustrated in Figure 1 has a training mode in which no medicament is actually released. Instead, during the training mode, the inhaler measures the patient's inhalation and provides feedback to the patient until the patient can achieve a correct inhalation profile defined by calibration data stored in the processing circuitry.

Examples of processing circuitry which may be used with the inhaler device 1 shown in Figure 1 will now be described.

### MDI processing circuitry 1

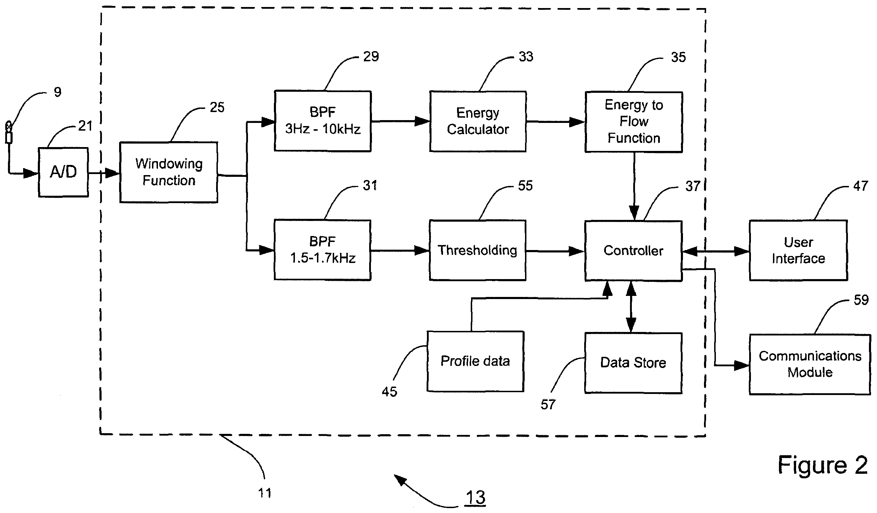

Figure 2 is a block diagram illustrating the main components of the electronic circuitry 13 used with the metered dose inhaler illustrated in Figure 1. As shown, the circuitry includes the microphone 9, the signals from which are input to an analogue to digital converter 21. The digitized samples obtained by the analogue to digital converter 21 are then input to a digital processor 11. As mentioned above, the processor 11 may be any suitably programmed microprocessor or ASIC based device.

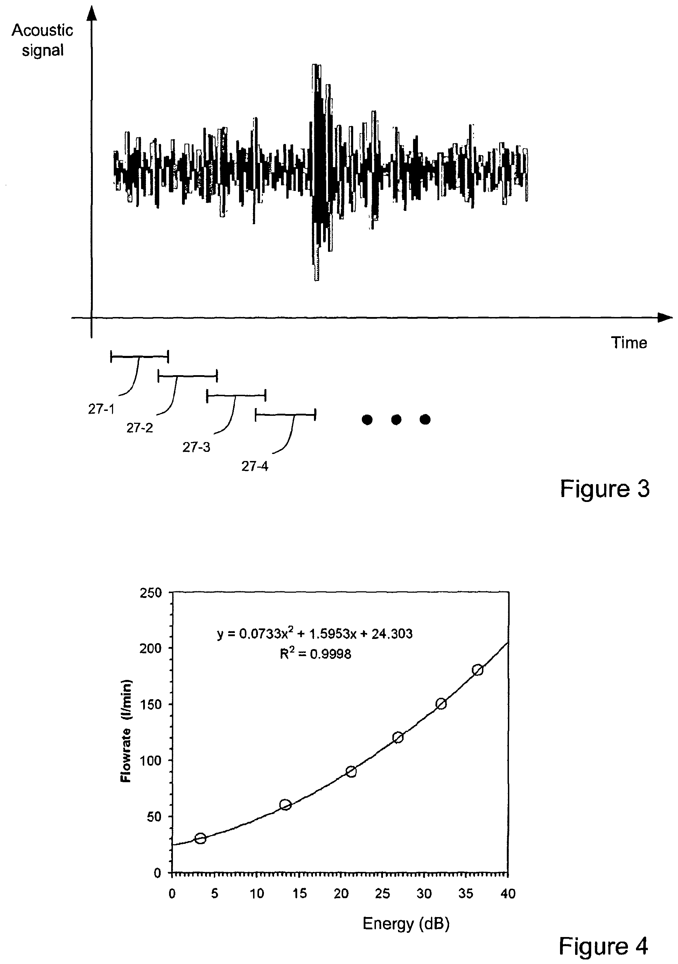

In this example, the functions performed by the processor 11 are illustrated as processing blocks. These processing blocks may be implemented either using hardware circuits but in this example are implemented as software routines run by the processor 11. Thus, as illustrated in Figure 2, the acoustic samples obtained from the analogue to digital converter 21 are firstly processed by a windowing function 25 which divides the samples into discrete blocks of samples by applying a suitable windowing function (such as a Hamming windowing) to reduce the effects of noise added by the windowing process. Figure 3 illustrates the windowing process and shows that the windowing function 25, in this example, extracts blocks 27-1, 27-2, 27-3, 27-4 of samples which partially overlap each other. In other examples and embodiments, the blocks 27 of samples may be non-overlapping. In this example, the acoustic signal from the microphone 9 is sampled at a sampling rate of 44.1 kHz and the windowing function 25 generates blocks of samples of 50ms duration at a rate of 22 blocks per second. Of course, other sampling rates and windowing rates may be used.

As illustrated in Figure 2, the blocks of samples output by the windowing function 25 are then passed to band pass filters 29 and 31. The band pass filter 29 is arranged to pass frequencies between 3Hz and 10kHz and to block other frequency components outside this range. The filtered samples are then passed to an energy calculator 33 which calculates the energy within each block 27 of samples. The energy value thus calculated is then passed to an energy to flow function 35 which determines the volumetric flow rate corresponding to the determined energy measure. In this example, the energy to flow function 35 is defined by a look up table which relates input energy values to corresponding flow rates. The look up table is calibrated in advance by drawing known flow rates through the inhaler and measuring the energy in the corresponding acoustic signal obtained from the microphone 9. The same look up table can be used in inhalers of the same design, although different look up tables will be required by inhalers having different acoustic characteristics.

Figure 4 is a plot illustrating the data obtained for the present inhaler 1 during the calibration process. The look up table used by the energy to flow function module 35 was determined from the data illustrated in Figure 4. As those skilled in the art will appreciate, instead of using a look up table to represent the measurements obtained during calibration, an equation, such as a quadratic function, may be used to define the relationship between the measured energy and the corresponding flow rate. The quadratic function for the plot illustrated in Figure 4 is also provided on the plot, where x is the measured energy for the current block 27 of samples and y is the corresponding determined flow rate.

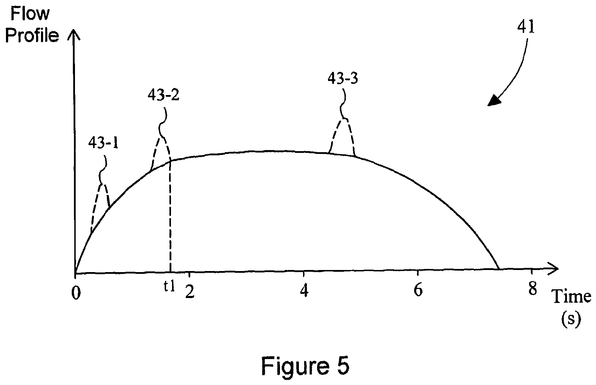

The flow rates determined by the energy to flow function 35 for the sequence of the blocks 27 of samples obtained during an inhalation are passed to the controller 37. The controller uses the determined flow rates to obtain a flow profile of the inhalation. Figure 5 schematically illustrates the resulting flow profile that is typically desirable for an MDI type inhaler. In particular, during an inhalation, it is typically desirable that the flow rate increases from zero to a maximum desired flow rate and remains at this maximum flow rate for a period of time before decreasing back to zero as the inhalation ends. The desired peak flow rate is usually much lower than that achievable by most users - and too strong an inhalation is one the many faults users have with using the inhaler.

During the above mentioned training procedure, the user simply inhales into the mouthpiece of the inhaler and the processing circuitry 11 determines the corresponding flow profile 41. If the user inhales strongly, then the peak of the flow profile will be too large which typically results in the medicament not being inhaled into the lungs but instead lining the back of the user's throat. Similarly, if the user inhales too softly and the peak of the flow profile 41 is too low, then the air flow may not be sufficient to draw the medicament into the user's lungs. Therefore, during the training mode, the controller 37 compares the obtained flow profile for each inhalation with stored flow profile data 45 (representing an ideal flow profile) and outputs indications to the user via a user interface 47 indicating whether or not the user is inhaling properly. The user interface 47 may include one or more LED lights or a display in order to output the information to the user. For example, the user interface 47 may include a green LED which is illuminated if the user inhales correctly and an amber LED which is illuminated if the user inhales incorrectly - for example that flashes quickly if the inhalation is too strong or that flashes slowly if the inhalation is too soft. Other possibilities are, of course, possible. The user interface 47 also includes a user input for allowing the user to set the inhaler 1 into the training mode discussed above and also to be able to return the inhaler 1 to its normal operating mode.

Figure 5 also shows three peaks 43-1, 43-2 and 43-3. These peaks 43 correspond to possible timings when the MDI canister 3 is fired. Ideally, the MDI canister 3 should be fired just before the time at which the flow rate of the inhalation peaks at time t₁. Therefore, if the MDI canister 3 is fired at the time corresponding to peak 43-1 then this is too early in the inhalation and may result in improper delivery of the medicament. Similarly, if the MDI canister 3 is fired at the time corresponding to the peak 43-3, then this is at a time well after the peak flow rate of the inhalation has been achieved and this may also result in the incorrect delivery of the medicament to the user.

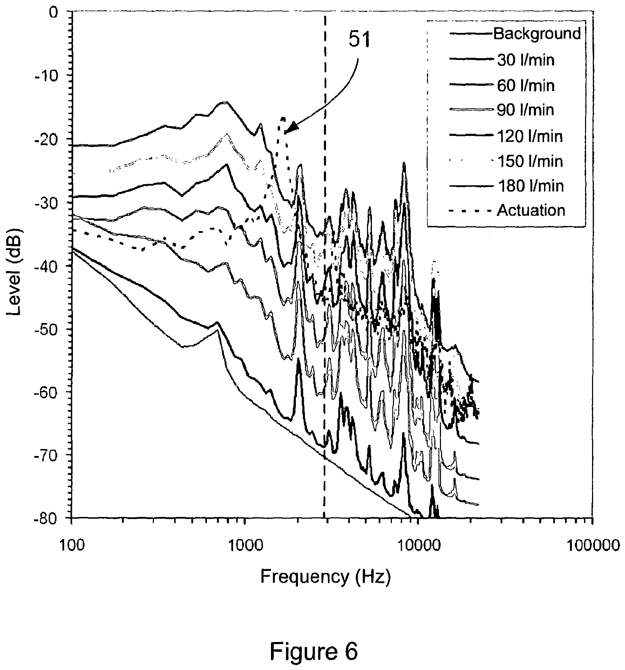

The inventors have found that the actuation (or firing) of the MDI canister 3 produces a notable peak in the spectrum of the acoustic signal at a frequency that is characteristic of the inhaler device (in the case of inhaler 1 shown in Figure 1 at a frequency of approximately 1.6kHz). Figure 6 illustrates the acoustic signal obtained for the inhaler show in Figure 1 for different flow rates and also showing the acoustic signal associated with the firing of the MDI canister 3 - as represented by the dashed plot 51. As can be seen from Figure 6, the actuation signal 51 has a peak at a frequency corresponding to approximately 1.6kHz which is clearly distinguishable from the other signals corresponding to the inhalation sound at different flow rates. Therefore, in order to detect the actuation signal 51, the processing circuitry 11 band pass filters the signal obtained from the microphone 9 using a band pass filter with a narrow pass-band centered around 1.6kHz. Referring to Figure 2, band pass filter 31 performs this narrow band filtering in order to extract the peak of the MDI actuation signal 51.

As shown in Figure 2, the output from the band pass filter 31 is input to a threshold module 55 which compares the filtered signal against a number of threshold values. In this example, two threshold values are used by the threshold module - a low threshold value and a high threshold value. The results of the thresholding performed by the thresholding module 55 are input to the controller 37. If the signal level output from the band pass filter 31 is below the low threshold value, then the controller 37 determines that no firing of the MDI canister 3 occurred in the current block 27 of samples. If the signal level is above the low threshold value but below the high threshold value, then the controller 37 uses this to identify a faulty firing - perhaps because the canister is nearly empty or because there is a partial blockage of the metering valve. If the signal level exceeds the high threshold value then the controller 37 determines that the MDI canister 3 did fire during the current block 27 of samples. Typically, the sound of the firing of the MDI canister 3 will last approximately 200ms and so the controller 37 should identify the firing of the MDI canister 3 within a number of consecutive blocks 27 of samples. Therefore, the controller 37 is able to detect accurately the timing at which the firing occurs and, by comparing this with the determined flow profile, can determine whether or not the firing has occurred too early or too late in the flow profile 41 or at a perfect timing, just before the peak of the inhalation flow profile 41. In this example, the controller 37 does this by integrating the flow profile 41 over the duration of the inhalation to determine the total displaced volume of air drawn by the inhalation and determines the ratio of the air drawn before the canister firing to the air drawn after the canister firing. If the ratio is above a first threshold, then the firing is too late and if the ratio is below a second lower threshold, then the firing was too early. If the ratio is between the two thresholds, then the controller determines that the firing occurred at the correct timing.

The controller 37 can then store the information obtained for each inhalation and canister firing in the data store 57. Alternatively, or in addition, the controller 37 can output the results of the processing and/or the measurements obtained to a communications module 59 for transmission to a remote device. The remote device may log the data which may then be viewed by a clinician and/or by a doctor or physician who can provide further instruction on correct usage of the inhaler. Similarly, the data stored in the data store 57 may be retrieved via the user interface 47 through an appropriate data reader. For example the user interface 47 may include a USB interface for allowing a computer device to connect to the controller 37 and hence to obtain the data stored in the data store 57. The way in which this can be achieved will be known to those skilled in the art.

During the above training mode, the controller 37 may also output indications (visual and/or audible) to the user as to whether they are pressing the canister 3 at the correct time during the inhalation. This will allow the user to get feedback or confirmation when they are using the inhaler correctly or otherwise. During the training mode, a training canister may be used that does not contain any medicament - thereby allowing the user to be able to practice using the device repeatedly without long periods between each use.

### Process flow charts

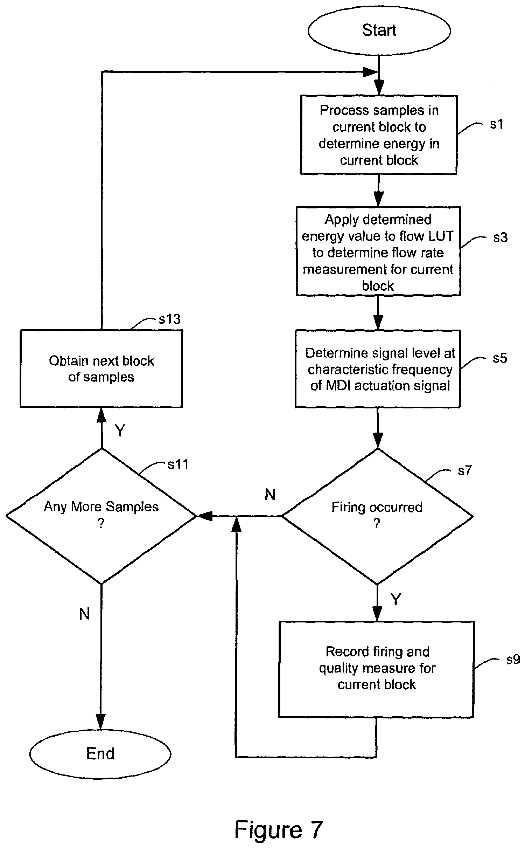

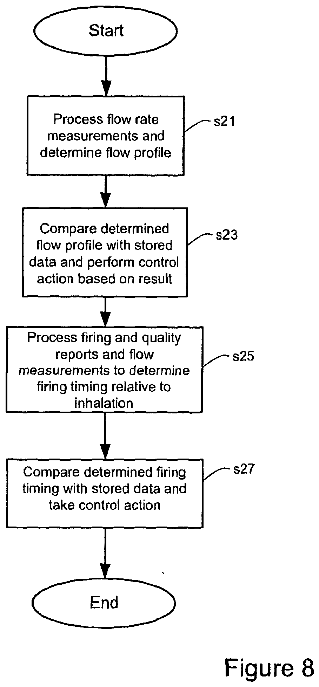

Figures 7 and 8 are flow charts illustrating the processing performed by the processing circuitry 11 illustrated in Figure 2. As shown, in step s1, the samples in the current block are processed to determine the energy of the signal within the current block 27. This determined energy value is then applied to the flow look up table (defined by the energy to flow function 35) to determine a flow rate measurement for the current block 27 of samples. In step s5, the signal level at the characteristic frequency of the MDI actuation signal (in this case at 1.6kHz) is determined. In step s7, a determination is made as to whether or not firing occurred by comparing the signal level at the characteristic frequency against the high and low thresholds of the thresholding module 55. If firing does occur, then at step s9 the fact that the firing has occurred is recorded and a quality measure of the firing is recorded based on whether or not the signal level is above or below the high threshold value. If firing does not occur or after recordation of the firing has been made, the processor proceeds to step s11 where a determination is made if there are any more samples to be processed. If there are no more samples (for example if a determination is made that the signal level drops below a defined minimum value) then the processing ends. Otherwise, the next block of samples is obtained in step s13 and the processing returns to step s1 where the same process is repeated for the next block of samples.

Figure 8 illustrates the processing performed by the controller 37 when processing the measurements and firing determinations obtained during a current inhalation.

In step s21, the flow rate measurements obtained for consecutive blocks of samples are processed to determine the flow profile 41 for the inhalation. This flow profile 41 is then compared with the stored flow profile data 45 to determine if the user has inhaled correctly and then an appropriate control action is taken depending on the result (for example the user may be signalled about the improper use of the device via the user interface 47). In step s25, the determined firing reports for the current inhalation are processed to determine the actual timing of the firing relative to the determined flow profile for the inhalation. In this way, spurious firing reports can be ignored and an accurate determination can be made as to exactly when the firing occurred. In step s27, the determined firing timing (relative to the flow profile 41) is compared with stored data (defining the optimum firing timing) and an appropriate control action is performed. For example, the timing information may simply be stored in the memory for subsequent use, or it may be transmitted to a remote location or it may be used to output an indication to the user as to whether or not the firing occurred at the correct timing during the inhalation.

### MDI processing circuitry 2

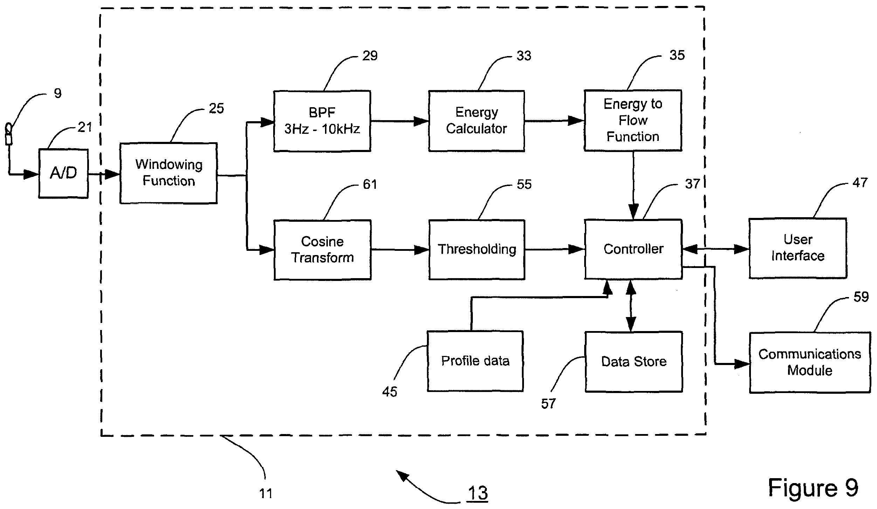

Figure 9 illustrates alternative processing circuitry 11 that can be used with the inhaler device 1 shown in Figure 1. As can be seen by comparing Figure 9 with Figure 2, the main difference, in this example, is that the band pass filter 31 is replaced with a cosine transform module 61. This cosine transform module 61 is programmed to calculate the cosine transform of the block 27 of samples at the characteristic frequency of the inhaler. With the inhaler illustrated in Figure 1, the characteristic frequency is 1.6kHz and therefore, the cosine transform module 61 only needs to calculate the cosine transform at this frequency. The output from the cosine transform module 61 represents the amplitude of the signal at the characteristic frequency. This amplitude value is then passed to the thresholding module 55 as before.

### DPI inhaler

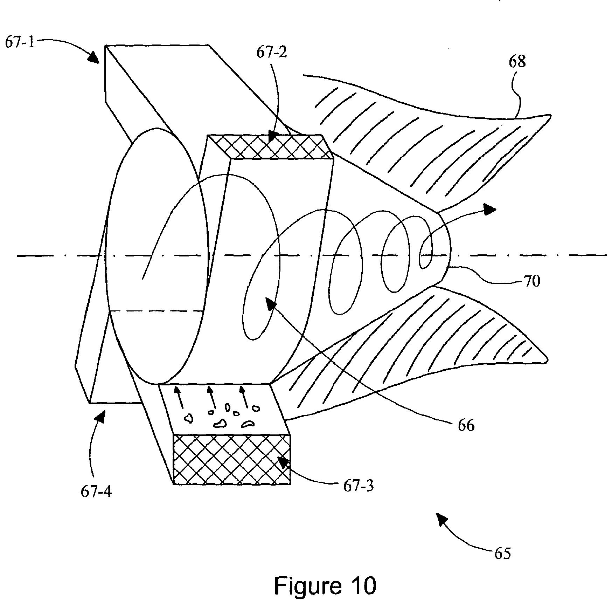

Figure 10 is a partially exploded view illustrating the main components of a dry powder inhaler (DPI) device 65. The DPI device 65 has a swirl chamber 66 having a plurality of tangential inlets 67-1 to 67-4 through which air is drawn when a user inhales through a mouthpiece 68 of the inhaler 65. During the inhalation process, a breath actuation mechanism (BAM) (not shown) is activated which releases the medicament into one of the inlets 67-3 and the active drug particles are deagglomerated from carriers (usually lactose) to create a free vortex within the swirl chamber 66. This swirling airflow is then concentrated through a smaller outlet 70 in the mouthpiece 68, increasing the tangential velocity of the airflow. This highly swirling airflow through the inhaler 65 produces a dominant acoustic frequency which is dependent upon the volumetric flow rate. Therefore, the inventors have found it is possible to determine the volumetric flow rate and other parameters (as will be described below) by performing a tonal analysis of the sound made by the inhaler 65.

### Exemplary DPI processing circuitry

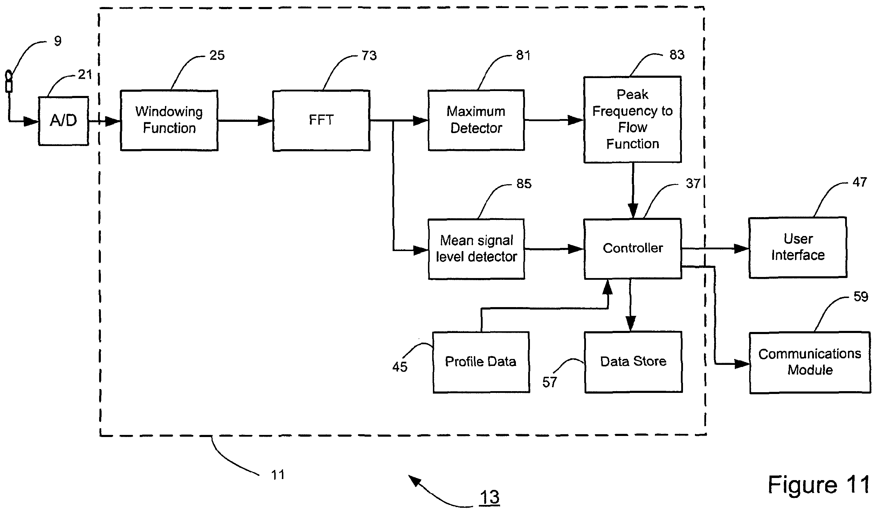

Figure 11 illustrates processing circuitry 11 used in this embodiment to determine the volumetric flow rate through the inhaler 65 shown in Figure 10 and used to detect events such actuation of the breath actuation mechanism (BAM) of the inhaler 65.

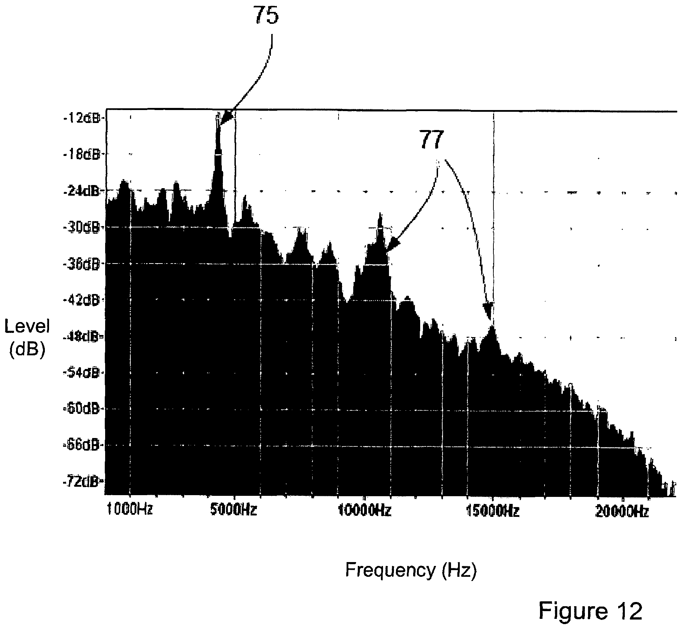

As shown in Figure 11, the acoustic signal picked up by the microphone 9 is converted into digital data by the analogue to digital converter 21 and the samples are then divided into blocks 27 of samples by the windowing function 25 (as per the first example). In this embodiment, each block 27 of samples is then passed to a Fast Fourier Transform (FFT) module 73 which performs a Fast Fourier Transform on each block 27 of samples individually. Figure 12 illustrates a typical FFT spectrum obtained by the FFT module 73. As shown, the spectrum for a block 27 of samples includes a dominant frequency component 75 resulting from the swirling airflow as well as other harmonic components 77.

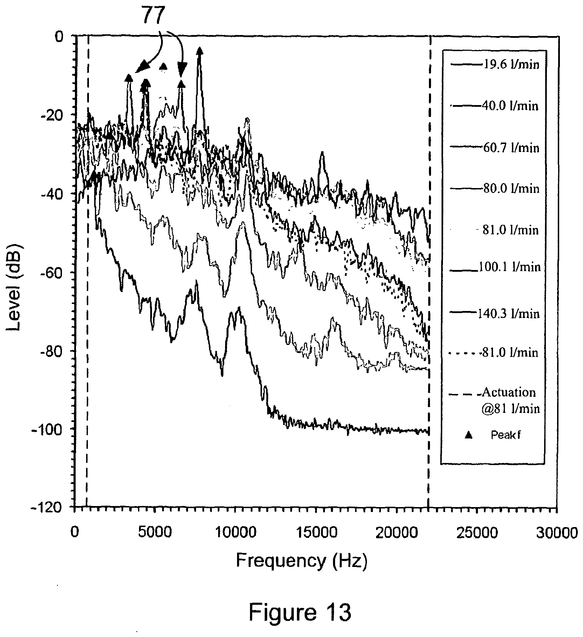

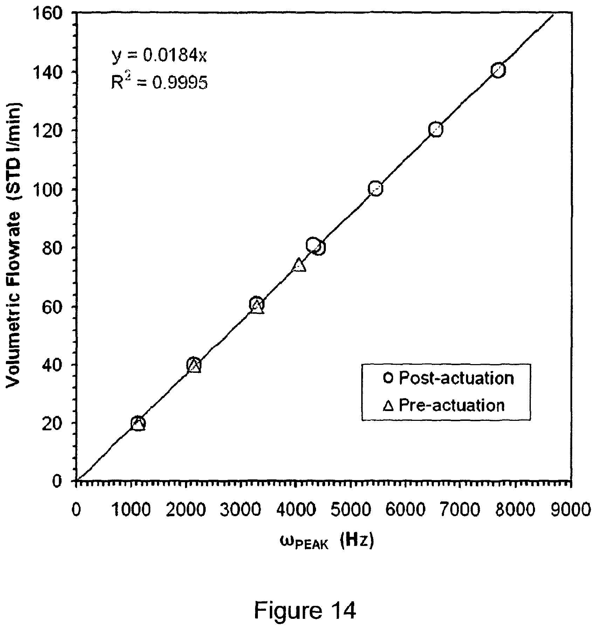

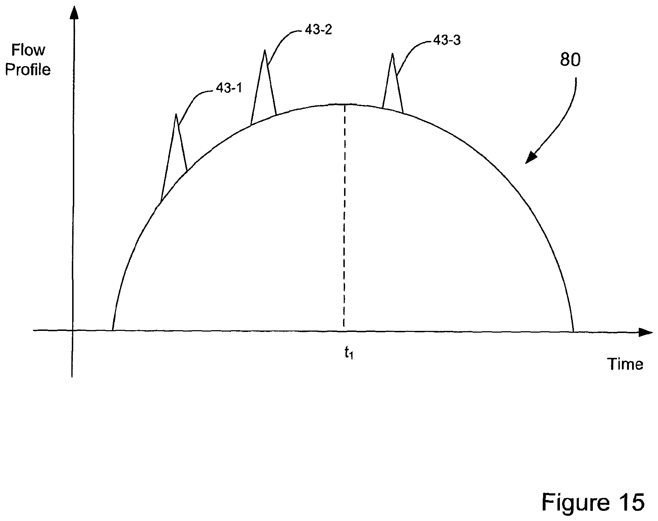

The inventors have found that the dominant frequency component 75 varies with the volumetric flow rate of air drawn through the inhaler. This is illustrated in the plot shown in Figure 13 which shows the spectrums obtained at different flow rates through the inhaler. As can be seen by the triangles 79 in Figure 13, the peak frequency changes with the flow rate. The inventors have found that for DPI type inhalers, the peak frequency varies approximately linearly (as shown in Figure 14) with the flow rate through the inhaler.
Therefore, as illustrated in Figure 11, the spectrum obtained from the FFT module 73 is input to a maximum detector 81 which identifies the frequency corresponding to the peak 75 in the spectrum. The determined frequency is then passed to a peak frequency to flow function module 83 which converts the peak frequency into a corresponding flow rate. This may be achieved using a look up table or using an equation corresponding to the function shown in Figure 14. The determined flow rate value for the current block of samples is then passed to the controller 37 for subsequent analysis. In particular, the controller 37 can use the determined flow rates to determine the overall flow profile for the inhalation and can use this information to train the user to use the inhaler correctly and/or can store the information or transmit it to a remote location as per the first example described above. The desired flow profile for a DPI inhaler is normally different to that shown in Figure 5 for an MDI. This is because, the energy within the user's inhalation is used to deagglomerate the medicament and so a shorter and stronger inhalation is typically required. A typical inhalation flow profile 80 for a DPI is illustrated in Figure 15. Figure 15 also shows possible timings for the firing of the BAM mechanism with the peaks 43. Ideally, the firing will occur just before the peak inhalation corresponding to peak 43-2 in Figure 15.

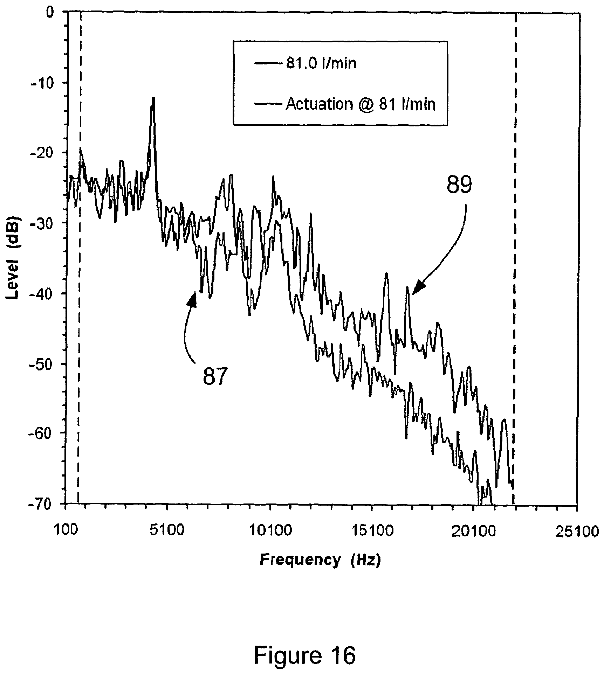

As mentioned above, a breath actuation mechanism (BAM) triggers when the user inhales through the mouthpiece, releasing the powdered medicament into the swirling airflow. The inventors have found that the actuation of the BAM does not affect the tonal characteristics of the spectrum obtained from the FFT module 73. However, the actuation of the BAM produces a loud "click" sound which is more predominant in the higher frequencies of the spectrum. This is illustrated in Figure 16 which shows a first spectrum 87 obtained from the FFT module 73 when a volumetric flow rate of 81 litres per minute is passing through the inhaler and a second spectrum 89 obtained with the same flow rate but at the instant in time when the BAM is activated. As can be seen from Figure 16, the signal level of the spectrum 89 is much higher for frequencies above 15kHz than in the corresponding spectrum 87 when the BAM is not activated.

Therefore, in this embodiment, the spectral output from the FFT module 73 is also input to a mean signal level detector 85 which determines the mean signal level of the spectrum in the higher frequency range (for example above 15kHz). The determined mean signal levels are then passed to the controller 37 which processes the received mean signals levels to determine whether or not the BAM has been activated. The inventors have found that this can be determined using a number of different techniques. For example, the controller 37 can consider the change in the mean signal level from one block of samples to the next. If the change in the mean signal level exceeds a predetermined threshold value (determined in advance during a calibration routine for the particular type of inhaler), then the controller 37 can infer that the BAM has been activated in the time period corresponding to the current block of samples being processed. As those skilled in the art will appreciate it is important to consider the difference between the mean signal levels in adjacent (or near adjacent) blocks 27 of samples in order to take into account the variation in the mean signal levels caused by the variation in the flow rates during the normal inhalation. In an alternative technique, calibration data may be stored in the inhaler 65 identifying typical mean signal levels for different flow rates. In this case, the controller 37 can use the flow rate determined by the peak frequency to flow function 83 for the current block 27 of samples, to determine from the calibration data what the corresponding mean signal level is for this flow rate. The controller 37 can then compare this calibration mean signal level with the mean signal level obtained from the mean signal level detector 85. If the mean signal level obtained from the detector 85 exceeds the calibration mean signal level by more than a predetermined amount, then the controller 37 can infer that the BAM has been actuated in the current block 27 of samples.

The inventors have also identified that a different sound is produced when the BAM is activated and dry powder is released into the swirl chamber 66 than when the BAM is activated and no powder is released into the swirl chamber 66. In particular, the plot shown in Figure 15 is for the case where the BAM 69 is activated and no powder is released into the swirl chamber 66. Therefore, the detection described above actually relates to detection of a misfiring of the inhaler. When powder is released into the swirl chamber 66 upon activation of the BAM 69, the peak frequency 75 described above temporarily drops in frequency. This is because the addition of the powder adds to the mass of the swirling air, which reduces the acoustic frequency of the swirl. However, this will actually make it easier to detect the activation of the BAM 69 using the second method described above as the reduction in the peak frequency will have the effect of lowering the flow rate determined by the peak frequency to flow function 83, which will in turn equate to a lower mean signal level determined using the calibration data. Therefore, by using different thresholds, the controller 37 is able to distinguish between the situation where the BAM 69 is activated and no dry powder is released into the swirl chamber 66 and the situation where the BAM 69 is activated and dry powder is released into the swirl chamber 66. Consequently, the controller 37 is able to detect the misuse or misfiring of the inhaler and output a warning to the user via the user interface 47 or is able to store the information for subsequent analysis or transmit the information to a remote source for immediate analysis.

In addition to and/or instead of processing the measurements received for each block 27 of samples, the controller 37 may also consider the measurements obtained during the entire inhalation. In this way, the controller 37 is better able to identify anomalies within the received measurements and detect events such as the firing of the BAM with or without the dry powder etc.

### Modifications and alternative embodiments

A number of embodiments have been described above that illustrate the way in which signals obtained from the microphone may be processed to determine various operational events during the use of an inhaler device. Various alternatives and modifications can be made to these embodiments and a number of these will now be described.

In the DPI embodiment described above, the processing circuitry was arranged to detect the dominant frequency component in the acoustic signal. This was achieved by performing a Fast Fourier Transform of the signal obtained from the microphone. In an alternative embodiment, the dominant frequency component may be determined using time domain techniques, for example by detecting zero crossings of the acoustic signal or by using banks of band pass filters and comparison circuits.

In the above embodiments, the processing electronics were arranged to detect the flow profile of the airflow drawn through the inhaler during the inhalation. As those skilled in the art will appreciate, other characteristics and metrics may be calculated. For example, the processing electronics may be arranged to calculate the inhaled volume, the peak inspiratory flow rate, the maximum lung capacity, the rate of change of inspiratory flow rate, the inhalation duration, the sustained average flow rate etc. Logging parameters such as these throughout the treatment period may provide valuable information about the efficacy of the treatment. Further, if for example, the peak inspiratory flow rate suddenly decreases two weeks into a one month prescription, the inhaler can flag a warning, prompting the user to call the doctor, or even communicating with the doctor directly via the communications module 59. As a further example, with DPI type inhalers, where the energy in the user's inhalation is used to aerosolise the medicament, an important parameter is the rate of change of the flow rate. An inhalation that has a large rate of change of flow rate before the peak inhalation is better for aerosolising the medicament that one having a low rate of change of flow rate. Therefore, measuring the rate of change of the flow rate can also be used to determine if the inhaler is used correctly.

In the embodiment described above, the flow rate was determined by determining the dominant frequency and relating this through stored calibration data to the flow rate. As the harmonics of the dominant frequency also vary with the flow rate, the processing electronics could be arranged to identify one or more of the harmonics as well or instead of the dominant frequency, and use these to determine the flow rate.

As an improvement to the embodiment, the energy in the acoustic signal may also be measured and used to determine a coarse measure of the flow rate (for example using the technique used in the first example). In particular, whilst the tonal analysis described above provides an accurate measure of the flow rate, it is most accurate for flow rates above about 20 l/min. Therefore, for lower flow rates, the flow rate can be determined using the energy in the acoustic signal. The energy signal may also be used as a check when the BAM actuates. In particular, as discussed above, when the BAM activates and medicament is added to the swirling air flow, this reduces the peak frequency which reduces the calculated flow rate. However, it is also possible that the user hiccupped during the inhalation and this is what caused the dip in the measured flow rate. By considering the measured flow rate using the energy measure, the processing electronics can distinguish between a drop in measured flow rate caused by a hiccup and a drop in measured flow rate caused by the firing of the BAM mechanism.

In the embodiments described above, the processing electronics determined the flow profile for the inhalation. In other embodiments, the processing electronics may only determine the flow profile for a part of the inhalation - for example the initial part until the drug has been released and the peak flow rate etc has been calculated.
In the above embodiment, the processing electronics were mounted in the inhalers. In an alternative embodiment, the processing may be performed by a remote processing device.

In such an embodiment, the inhaler would record the signals obtained from the microphone and the data stored in the inhaler would then be downloaded to a computer device to perform the processing in the manner described above.

In the above embodiments, the processing electronics is able to process the signal obtained from the microphone and detect if the delivery mechanism is activated during the inhalation and, if it is, to detect if the drug is also delivered by the mechanism. The processing electronics may maintain a count of the number of times that the delivery device is activated and the drug is successfully delivered and the number of times that the delivery device is activated but no drug is delivered. This information may be useful for subsequent diagnosis by the clinician or physician. Additionally, real time feedback may also be provided to the user so that they know if the drug was actually delivered. Very often with inhaler devices, users take too much of the drug because they do not realise that the drug is dispensed during one or more of their inhalations. This problem can thus be solved by this inhaler device.

Some inhalation devices that are existing in the market have to be disassembled in order to clean the inhaler. After cleaning, the inhaler must then be reassembled before it can be used again. Because the processing circuitry includes calibration data relating to the tonal characteristics of the inhaler, the processing circuitry can detect if the device is reassembled incorrectly. In particular, if the device is incorrectly assembled then this will change the acoustic characteristics of the inhaler. The processing circuitry can detect these changes in the acoustic characteristics and can therefore output a warning to the user indicating that the device has not been reassembled properly. For example, with the DPI type of inhaler described above, if the device is not re-assembled correctly, then no swirling airflow may be produced. Therefore, if no tonal response is obtained yet an energy analysis indicates flow through the inhaler, then the controller can infer that the inhaler has been assembled incorrectly.

Similarly, if the inhaler is intended to work with a spacer and/or a holding chamber, removing the spacer or the holding chamber will change the acoustic characteristics of the inhaler and this can be detected by the processing electronics and a warning given or data recorded relating to the detected missing component.

Some inhaler devices that are provided include a mechanical counter that increments each time the drug is dispensed. Such mechanical counters typically make a clicking sound when they change value and this can also be detected by the processing circuitry.

Some dry powder inhalers use capsules to store the drug and the capsules have a foil which has to be pierced before the drug can be delivered. In such embodiments, the sound made by the piercing of the foil may be detected by the microphone and the processing electronics may detect the time when the foil is pierced. This time may then be recorded together with the time that the drug is subsequently delivered so that information about the time gap between piercing the foil and drug delivery can be determined. This information may be important, for example, if it is known that the drug deteriorates once the foil has been pierced and the drug is open in contact with the atmosphere.

Many of the existing inhalers include a cap to cover the mouthpiece. This is to prevent the ingress of dirt and dust which may block the delivery mechanism. Typically, the removal of the cap and the replacement of the cap on the inhaler makes a sound. The sound of the removal of the cap and the sound of the replacement of the cap may be detected by the processing electronics and used, for example, to output a warning to the user if the cap is removed for longer than a predetermined time. For example, an audible warning may be sounded if the user does not replace the cap after a predetermined time.

Inhaler devices like the ones described above are often dropped or knocked which may damage the device. The processing circuitry may be arranged to detect loud noises caused by, for example, dropping the inhaler and to output a warning to the user and/or to a clinician so that the inhaler device can be replaced.

Many inhaler devices are supposed to be shaken before being used. In one embodiment, the inhaler device also includes an accelerometer for sensing the shaking of the device prior to use. A proximity sensor may also be added to the inhaler so that the inhaler can distinguish shaking of the inhaler in a bag (accidental shaking) from shaking by the user when they are holding the device prior to use of the inhaler. Again, if the controller detects incorrect shaking, then a warning can be output to the user or signalled to a doctor or clinician.

In the above embodiments, the microphone was placed inside the air channel of the inhaler in order to maximise the signal levels and frequency response detected by the electronics. However, in an alternative embodiment, the microphone may be placed behind the wall defining the flow channel. However, this is not preferred as even 1 mm of plastic will attenuate the sound and, in particular, the high frequencies, thereby reducing the sensitivity of the electronics to detect events accurately.

These and various other modifications and alternatives will be apparent to those skilled in the art.

## Claims

1. Processing circuitry (13) for use in processing signals obtained from a microphone (9) mounted on a body of a drug delivery device (1) to determine operating conditions of the drug delivery device (65), the processing circuitry (13) being **characterised by**:
means (73, 81) for determining a frequency of a dominant frequency component or a harmonic thereof in the acoustic signal received from the microphone (9); and
means (83) for converting the determined frequency of the dominant frequency component or the harmonic thereof into a flow rate value using stored calibration data.

2. Processing circuitry according to claim 1, operable to track the dominant or harmonic frequency in the acoustic signal to determine a flow profile for at least a part of the inhalation.

3. Processing circuitry according to claim 2, further comprising:
means (37) for processing the signal obtained from the microphone (9) to detect the timing of the delivery of a medicament during the inhalation.

4. Processing circuitry according to claim 3, further comprising:
means (37) for comparing the detected timing of said delivery relative to said flow profile with stored second calibration data to determine if the delivery of the medicament meets a desired delivery condition.

5. Processing circuitry according to claim 3 or 4, wherein said means (37) for detecting said timing comprises means (37) for detecting a change in the mean signal level of at least a portion of the signal obtained from the microphone (9).

6. Processing circuitry according to claim 5, wherein said means (37) for detecting a change in the mean signal level detects the change in the mean signal level for an upper frequency band of the signal obtained from the microphone.

7. Processing circuitry according to any of claims 1 to 6, wherein the means (73, 81) for determining said dominant or harmonic frequency comprises means (73) for determining a spectrum of the signal obtained from the microphone (9) and means (81) for processing the spectrum to identify said dominant or harmonic frequency.

8. Processing circuitry according to any of claims 1 to 7, further comprising means (29, 33, 35) for determining the energy within the signal obtained from the microphone (9) and for determining a flow rate measurement from the determined energy and stored calibration data.

9. Processing circuitry according to claim 8, wherein the means for determining the energy within the signal comprises a band pass filter (29) for filtering the signal obtained from the microphone and is arranged to determine the energy within the filtered signal.

10. Processing circuitry according to any preceding claim, wherein the means (73, 81) for determining the frequency of said dominant frequency component or of said harmonic thereof is arranged to use time domain based analysis techniques on the acoustic signal from the microphone (9).

11. Processing circuitry according to any preceding claim, wherein said means (83) for converting is arranged to use calibration data in the form of a look-up table or in the form of an equation to convert the determined frequency of the dominant frequency component or the harmonic thereof into a flow rate value.

12. A drug delivery system comprising:
a drug delivery device (65) having a body with a mouthpiece and a microphone (9) mounted on the body for sensing sounds made by the drug delivery device (65) during operation; and
processing circuitry (13) according to any of claims 1 to 10 operable to process the signal obtained from the microphone (9) to determine operating conditions of the drug delivery device (65).

13. A system according to claim 12, wherein the processing circuitry is mounted within the body of the drug delivery device (65).

14. A system according to claim 12 or 13, wherein the drug delivery device (65) is a metered dose inhaler or a dry powder inhaler.

15. A computer implementable instructions product comprising computer implementable instructions for causing a programmable processor device to become configured as the processing circuitry according to any of claims 1 to 11.

## Patentansprüche

1. Verarbeitungsschaltung (13) zur Verwendung bei der Verarbeitung von Signalen, die von einem Mikrofon (9) erhalten werden, das an einem Körper einer Arzneimittelzuführungsvorrichtung (1) angebracht ist, um die Betriebsbedingungen der Arzneimittelzuführungsvorrichtung (65) zu bestimmen, wobei die Verarbeitungsschaltung (13) durch Folgendes gekennzeichnet ist:
ein Mittel (73, 81) zum Bestimmen einer Frequenz einer dominanten Frequenzkomponente oder einer Harmonischen davon in dem von dem Mikrofon (9) empfangenen akustischen Signal; und
ein Mittel (83) zum Umwandeln der bestimmten Frequenz der dominanten Frequenzkomponente oder der Harmonischen davon in einen Strömungsratenwert unter Verwendung gespeicherter Kalibrierungsdaten.

2. Verarbeitungsschaltung nach Anspruch 1, die in der Lage ist, die dominante oder harmonische Frequenz in dem akustischen Signal zu verfolgen, um ein Strömungsprofil für mindestens einen Teil der Inhalation zu bestimmen.

3. Verarbeitungsschaltung nach Anspruch 2, die ferner Folgendes umfasst:
ein Mittel (37) zum Verarbeiten des von dem Mikrophon (9) erhaltenen Signals, um den Zeitpunkt der Zuführung eines Medikaments während der Inhalation zu erfassen.

4. Verarbeitungsschaltung nach Anspruch 3, die ferner Folgendes umfasst:
ein Mittel (37) zum Vergleichen des erfassten Zeitablaufs der genannten Zuführung im Verhältnis zum genannten Strömungsprofil mit gespeicherten zweiten Kalibrierungsdaten, um festzustellen, ob die Zuführung des Arzneimittels einen gewünschten Zuführungszustand erfüllt.

5. Verarbeitungsschaltung nach Anspruch 3 oder 4, wobei die genannte Vorrichtung (37) zum Erfassen des genannten Zeitablaufs Mittel (37) zum Erfassen einer Änderung des mittleren Signalpegels von mindestens einem Teil des von dem Mikrofon (9) erhaltenen Signals aufweist.

6. Verarbeitungsschaltung nach Anspruch 5, wobei das genannte Mittel (37) zum Erfassen einer Änderung im mittleren Signalpegel die Änderung im mittleren Signalpegel für ein oberes Frequenzband des Signals erfasst, das von dem Mikrophon erhalten wird.

7. Verarbeitungsschaltung nach einem der Ansprüche 1 bis 6, wobei das Mittel (73, 81) zum Bestimmen der genannten dominanten oder harmonischen Frequenz Folgendes umfasst: ein Mittel (73) zum Bestimmen eines Spektrums des Signals, das von dem Mikrofon (9) erhalten wird, und ein Mittel (81) zum Verarbeiten des Spektrums, um die genannte dominante oder harmonische Frequenz zu identifizieren.

8. Verarbeitungsschaltung nach einem der Ansprüche 1 bis 7, die ferner ein Mittel (29, 33, 35) zum Bestimmen der Energie innerhalb des Signals, das von dem Mikrofon (9) erhalten wird, und zum Bestimmen einer Strömungsratenmessung aus der ermittelten Energie und den gespeicherten Kalibrierungsdaten umfasst.

9. Verarbeitungsschaltung nach Anspruch 8, wobei das Mittel zum Bestimmen der Energie innerhalb des Signals ein Bandpassfilter (29) zum Filtern des Signals umfasst, das von dem Mikrophon erhalten wird, und dafür ausgelegt ist, die Energie innerhalb des gefilterten Signals zu bestimmen.

10. Verarbeitungsschaltung nach einem der vorhergehenden Ansprüche, wobei das Mittel (73, 81) zum Bestimmen der Frequenz der genannten dominanten Frequenzkomponente oder der genannten Harmonischen davon dafür ausgelegt ist, zeitdomänenbasierte Analyseverfahren für das akustische Signal von dem Mikrofon (9) anzuwenden.

11. Verarbeitungsschaltung nach einem der vorhergehenden Ansprüche, wobei das genannte Mittel (83) zum Umwandeln dafür ausgelegt ist, Kalibrierungsdaten in Form einer Lookup-Tabelle oder in Form einer Gleichung zu verwenden, um die bestimmte Frequenz der dominanten Frequenzkomponente oder der Harmonischen davon in einen Strömungsratenwert umzuwandeln.

12. Arzneimittelzuführungssystem, das Folgendes umfasst:
eine Arzneimittelzuführungsvorrichtung (65), die einen Körper mit einem Mundstück und ein Mikrofon (9), das an dem Körper angebracht ist, um Geräusche zu erfassen, die von der Arzneimittelzuführungsvorrichtung (65) während des Betriebs erzeugt werden, aufweist; und
eine Verarbeitungsschaltung (13) nach einem der Ansprüche 1 bis 10, die dafür ausgelegt ist, das Signal zu verarbeiten, das von dem Mikrofon (9) erhalten wurde, um die Betriebsbedingungen der Arzneimittelzuführungsvorrichtung (65) zu bestimmen.

13. System nach Anspruch 12, wobei die Verarbeitungsschaltung in dem Körper der Arzneimittelzuführungsvorrichtung (65) angebracht ist.

14. System nach Anspruch 12 oder 13, wobei die Arzneimittelzuführungsvorrichtung (65) ein Dosierinhalators oder ein Trockenpulverinhalator ist.

15. Produkt für computerimplementierbare Anweisungen, das computerimplementierbare Anweisungen umfasst, um zu bewirken, dass ein programmierbares Prozessorgerät als Verarbeitungsschaltung nach einem der Ansprüche 1 bis 11 konfiguriert wird.

## Revendications

1. Circuits de traitement (13) destinés à être utilisés dans le traitement de signaux obtenus à partir d'un microphone (9) monté sur un corps d'un dispositif d'administration de médicaments (1) afin de déterminer des conditions de fonctionnement du dispositif d'administration de médicaments (65), les circuits de traitement (13) étant **caractérisés par** :
des moyens (73, 81) pour déterminer une fréquence d'un composant de fréquence dominante ou une harmonique de celle-ci dans le signal acoustique reçu à partir du microphone (9) ; et
des moyens (83) pour convertir la fréquence déterminée du composant de fréquence dominante ou l'harmonique de celle-ci en une valeur de débit grâce à l'utilisation de données d'étalonnage stockées.

2. Circuits de traitement selon la revendication 1, exploitables de façon à assurer le suivi de la fréquence dominante ou harmonique dans le signal acoustique afin de déterminer un profil d'écoulement pour au moins une partie de l'inhalation.

3. Circuits de traitement selon la revendication 2, comprenant en outre :
des moyens (37) pour traiter le signal obtenu à partir du microphone (9) afin de détecter la synchronisation de l'administration d'un médicament au cours de l'inhalation.

4. Circuits de traitement selon la revendication 3, comprenant en outre :
des moyens (37) pour comparer la synchronisation détectée de ladite administration par rapport audit profil d'écoulement avec des deuxièmes données d'étalonnage stockées afin de déterminer si l'administration du médicament satisfait à une condition d'administration désirée.

5. Circuits de traitement selon la revendication 3 ou 4, lesdits moyens (37) pour détecter ladite synchronisation comprenant des moyens (37) pour détecter un changement dans le niveau de signal moyen d'au moins une portion du signal obtenu à partir du microphone (9).

6. Circuits de traitement selon la revendication 5, lesdits moyens (37) pour détecter un changement dans le niveau de signal moyen détectant le changement dans le niveau de signal moyen pour une bande de fréquence supérieure du signal obtenu à partir du microphone.

7. Circuits de traitement selon l'une quelconque des revendications 1 à 6, les moyens (73, 81) pour déterminer ladite fréquence dominante ou harmonique comprenant des moyens (73) pour déterminer un spectre du signal obtenu à partir du microphone (9), et des moyens (81) pour traiter le spectre afin d'identifier ladite fréquence dominante ou harmonique.

8. Circuits de traitement selon l'une quelconque des revendications 1 à 7, comprenant en outre des moyens (29, 33, 35) pour déterminer l'énergie au sein du signal obtenu à partir du microphone (9) et pour déterminer une mesure du débit à partir de l'énergie déterminée et de données d'étalonnage stockées.

9. Circuits de traitement selon la revendication 8, les moyens pour déterminer l'énergie au sein du signal comprenant un filtre passe-bande (29) pour filtrer le signal obtenu à partir du microphone et étant agencés de façon à déterminer l'énergie au sein du signal filtré.

10. Circuits de traitement selon une quelconque revendication précédente, les moyens (73, 81) pour déterminer la fréquence dudit composant de fréquence dominante ou de ladite harmonique de celle-ci étant agencés de façon à utiliser des techniques d'analyse basées sur le domaine temporel sur le signal acoustique en provenance du microphone (9).

11. Circuits de traitement selon une quelconque revendication précédente, lesdits moyens (83) pour la conversion étant agencés de façon à utiliser des données d'étalonnage sous la forme d'une table de consultation ou sous la forme d'une équation afin de convertir la fréquence déterminée du composant de fréquence dominante ou l'harmonique de celle-ci en une valeur de débit.

12. Système d'administration de médicaments comprenant :
un dispositif d'administration de médicaments (65) possédant un corps avec un embout buccal et un microphone (9) monté sur le corps pour capter des sons produits par le dispositif d'administration de médicaments (65) au cours du fonctionnement ; et
des circuits de traitement (13) selon l'une quelconque des revendications 1 à 10 exploitables de façon à traiter le signal obtenu à partir du microphone (9) afin de déterminer des conditions d'exploitation du dispositif d'administration de médicaments (65).

13. Système selon la revendication 12, les circuits de traitement étant montés à l'intérieur du corps du dispositif d'administration de médicaments (65).

14. Système selon la revendication 12 ou 13, le dispositif d'administration de médicaments (65) étant un inhalateur à dose mesurée ou un inhalateur à poudre sèche.

15. Produit à instructions apte à être mis en oeuvre par ordinateur, comprenant des instructions aptes à être mises en oeuvre par ordinateur afin d'obliger un dispositif à processeur programmable à se laisser configurer en tant que circuits de traitement, selon l'une quelconque des revendications 1 à 11.
